# FASCICULE DE BREVET EUROPEEN

(11) **EP 4 463 114 B1**
(45) Date de publication et mention de la délivrance du brevet: **12.08.2026**
(21) Numéro de dépôt: 23703263.6
(22) Date de dépôt: 05.01.2023
(51) Int. Cl.: A61F 9/00, A61J 1/14

(54) **FLACON MULTIDOSES CONTENANT UN PRODUIT OPHTALMIQUE VISQUEUX**
MEHRFACHDOSIERUNGSSPENDER MIT EINEM VISKOSEN OPHTHALMISCHEN PRODUKT
MULTIDOSE DISPENSER CONTAINING A VISCOUS OPHTHALMIC PRODUCT

(30) Priorité: 10.01.2022 FR 2200158
(43) Date de publication de la demande: 20.11.2024
(73) Titulaire: Laboratoires THEA, 63100 Clermont-Ferrand (FR)
(72) Inventeur: QUAGLIA, Benjamin, 63119 Châteaugay (FR)
(74) Mandataire: Santarelli
(86) Numéro de dépôt international: PCT/FR2023/050012
(87) Numéro de publication internationale: WO 2023/131760

(56) Documents cités:
- EP-A1- 1 593 605
- WO-A1-2012/013894
- US-A- 6 105 828

## Description

La présente invention porte sur un flacon multidoses qui contient un produit ophtalmique visqueux stérile et sans conservateur, et qui est adapté au conditionnement, au stockage et à la distribution goutte à goutte du produit ophtalmique visqueux qu'il contient.

Dans le domaine des compositions pharmaceutiques et en particulier des produits ophtalmiques, il existe de nombreux produits destinés à être déposés goutte à goutte à la surface de l'œil. Ces produits sont bien souvent liquides. Néanmoins, l'utilisation de produits ophtalmiques visqueux se développe. En effet, certaines compositions ophtalmiques se révèlent plus efficaces lorsqu'elles sont délivrées sous la forme d'un fluide visqueux, elles couvrent mieux la surface de l'œil et/ou demeurent plus longtemps en contact avec l'œil, ce qui améliore l'efficacité du produit et la tolérance locale à ce produit.

Par « visqueux » (notamment dans les expressions « produit visqueux », « fluide visqueux », « compositions visqueuses », gels, etc) il est entendu que la viscosité est supérieure ou égale à 200 cP. Les produits ophtalmiques visqueux visés dans le cadre de la présente invention sont généralement envisagés avec une viscosité supérieure à 500cP, et allant jusque 1500 cP, afin d'être administrés sous forme de gouttes.

Les produits considérés visqueux dans le présent document peuvent être Newtoniens ou non-Newtoniens. Les valeurs indiquées sont obtenues selon la méthode du viscosimètre rotatif telle que définie dans la Pharmacopée européenne 6.0 (01/2008).

Les produits ophtalmiques sont utilisés par petites doses, d'une ou quelques gouttes issues d'un flacon lors de chaque utilisation, alors que l'on doit conserver le reste du produit contenu dans le flacon pendant un certain temps, généralement plusieurs jours voire plusieurs semaines. Le produit contenu dans le flacon doit généralement être maintenu à l'abri de toute contamination environnementale et/ou microbiologique par des agents bactériens ou autres et provenant de l'extérieur du flacon.

Il existe en outre un intérêt particulier de proposer des solutions ophtalmiques sans conservateur. En effet, les produits utilisés en tant que conservateur dans les solutions ophtalmiques, pour assurer la stérilité des produits ophtalmiques vis-à-vis des bactéries et des champignons, peuvent avoir des effets indésirables importants.

Les conservateurs, notamment le chlorure de benzalkonium, sont irritants pour les yeux et peuvent altérer le film lacrymal, et peuvent provoquer une atteinte tissulaire par apoptose. L'utilisation à long terme de conservateurs a des conséquences indésirables sur l'œil.

Ainsi, l'utilisation de produits ophtalmiques sans conservateurs est bien souvent recommandable.

Une première solution pour conserver la stérilité de tels produits ophtalmiques sans conservateur consiste à les conditionner en doses uniques, aussi appelées « unidoses ». Le conditionnement en unidose n'est cependant pas applicable à tous les produits, et génère de nombreux déchets, le conditionnement de chaque dose devant être jeté après l'utilisation. Pour un traitement de longue durée, le conditionnement en unidose n'apparait ainsi pas approprié.

Dans ce contexte, des flacons contenant plusieurs doses, ou flacons « multidoses », configurés pour assurer la stérilité du produit qu'ils contiennent, y compris après ouverture et délivrance d'une première dose, ont été développés. Ces flacons ont pour but de conserver la stérilité dans le flacon tant que dure la consommation du produit jusqu'à l'épuisement complet du contenu du flacon.

Par exemple, le document FR2816600 divulgue un flacon de type goutte à goutte, dont le réservoir comporte une paroi déformable permettant de créer une surpression entrainant le passage du produit contenu au travers d'une membrane stérilisante. La surface de cette membrane est partiellement hydrophile et partiellement hydrophobe, et plus particulièrement sélectivement perméable aux liquides aqueux en sa partie hydrophile et sélectivement perméable à l'air en sa partie hydrophobe. Ainsi, le liquide traverse la membrane en sa partie hydrophile lors de sa distribution avant d'atteindre un embout de distribution goutte à goutte, tandis que de l'air peut être aspiré dans le flacon au travers de la partie hydrophobe pour compenser le volume de liquide distribué lorsque le réservoir n'est plus comprimé et revient à sa forme initiale. Un tampon poreux permet une régulation du flux dans le flacon, nécessaire à la bonne formation des gouttes.

Ce type de flacon est pleinement satisfaisant pour conditionner et distribuer de nombreux produits stériles sans conservateur, et notamment des produits ophtalmiques liquides sans conservateur.

Néanmoins, ce flacon n'est pas adapté à la distribution de certains produits. En particulier, les produits ophtalmiques visqueux ne peuvent pas être distribués de façon satisfaisante par ce type de flacon. En effet, du fait de la faible porosité de la membrane, les produits visqueux ne peuvent pas la traverser, ou mal. Pour traverser la membrane, plus le produit est visqueux, plus le produit doit être mis en pression. Le risque de rupture de la membrane est alors important. Pour les produits les plus visqueux, la distribution est tout simplement impossible.

De manière générale, lorsqu'un produit visqueux est conditionné dans un flacon multidoses à paroi déformable, le produit visqueux tend à adhérer aux parois et au fond du flacon. En particulier, entre deux distributions, le produit retombe au fond du flacon, et n'est plus en contact avec l'embout de distribution. Lors d'une distribution suivante de produit, le flacon est retourné mais le produit adhère au fond du flacon et ne vient pas immédiatement au contact de l'embout de distribution. La déformation de la paroi entraîne ainsi en premier lieu l'expulsion de l'air présent dans le flacon, et, au fur et à mesure des distributions, il est nécessaire de déformer de plus en plus le flacon pour provoquer une distribution de produit, ce qui peut d'ailleurs endommager le flacon. L'adhésion du produit aux parois du flacon rend complexe voire impossible la délivrance de tout le produit présent dans le flacon, ce qui n'est pas souhaitable pour un produit ophtalmique, qui est conditionné pour garantir un nombre de gouttes précis et qui a généralement une grande valeur.

Le document EP1985543 présente un flacon pour produit ophtalmique. Le réservoir du flacon est du type « à délaminage », ce qui signifie que la couche interne du réservoir peut se séparer du reste des parois pour former une poche interne déformable. Ce flacon comporte une valve anti-retour traversée par le produit sortant du flacon et comporte, en aval de cette valve, un filtre permettant le filtrage de certaines bactéries. Ce flacon n'est cependant pas adapté à délivrer un produit visqueux et/ou à garantir la stérilité du produit délivré.

Le document EP1593605 a un enseignement similaire.

La présente invention vise à proposer un flacon multidoses, contenant un produit ophtalmique visqueux, stérile et sans conservateur, qui résout tout ou partie des problèmes évoqués ci-dessus.

Ainsi, l'invention porte sur un flacon contenant un produit ophtalmique visqueux. Comme indiqué ci-avant, il s'agit de produits ayant une viscosité supérieure ou égale à 200 cP. Le flacon comporte un réservoir comportant une paroi définissant un volume interne et qui est adaptée à se déformer sous l'effet d'une pression exercée sur le réservoir par un utilisateur du flacon et à reprendre spontanément sa forme d'origine après relâchement de ladite pression. Le produit ophtalmique visqueux contenu dans le flacon est stérile et sans conservateur. Le réservoir comporte en outre une poche souple dans le volume interne défini par la paroi. La poche souple contient le produit ophtalmique visqueux.

Le flacon comporte en outre :
- un moyen d'entrée d'air entre la paroi du réservoir et la poche souple,
- une tête rapportée sur un col du réservoir et comportant un embout goutte à goutte comportant un orifice de distribution, et
- une valve unidirectionnelle interposée entre le réservoir et l'orifice de distribution, la valve unidirectionnelle étant configurée pour empêcher l'entrée d'air provenant de l'extérieur du flacon dans le réservoir. Ainsi, suite à une distribution de produit ophtalmique, le volume de produit ophtalmique distribué est compensé par l'entrée d'un volume d'air correspondant entre la paroi du réservoir et la poche souple via le moyen d'entrée d'air.

Le flacon comporte des surfaces internes situées entre la valve unidirectionnelle et l'orifice de distribution, et la totalité de ces surfaces internes portent un traitement antibactérien et/ou un traitement qui en diminue la mouillabilité.

Par « la totalité des surfaces internes » il est en particulier entendu toutes les surfaces exposées au produit ophtalmique lors de sa sortie du flacon.

La présente invention propose ainsi un flacon de distribution goutte à goutte contenant un produit ophtalmique visqueux, qui est parfaitement adapté à la distribution d'un tel produit. La viscosité est du produit contenue dans le flacon est mesurée selon la méthode du viscosimètre rotatif telle que définie dans la Pharmacopée européenne 6.0 (01/2008). Il est notable que cette méthode n'a pas évolué à ce jour dans la Pharmacopée européenne, il est donc possible de se référer à la Pharmacopée européenne de la version 6.0 à la version 10.6 en vigueur à ce jour.

Le flacon proposé est d'utilisation simple pour l'utilisateur, en ce qu'il permet la distribution de produit par simple pression sur le réservoir, ce qui est intuitif.

En outre, la poche souple qui est présente dans le volume interne défini par la paroi du réservoir permet une distribution complète du produit qu'elle contient, c'est-à-dire que toutes les doses de produit contenues dans la poche souple peuvent être effectivement distribuées.

La distribution de produit peut être également réalisée tandis que le flacon est positionné selon diverses orientations. En effet, au fur et à mesure que des doses de produit sont distribuées, le volume de produit distribué est compensé par l'entrée d'un volume équivalent d'air entre la paroi déformable du réservoir et la poche souple. Le volume de la poche souple s'adapte à celui du produit qu'elle contient. Le produit contenu dans la poche souple demeure ainsi à proximité immédiate de la tête de distribution, et aucune bulle d'air ne se forme dans la poche souple. Cela est particulièrement important dans la mesure où le produit contenu dans le flacon est visqueux, et donc susceptible d'adhérer aux parois du flacon. La mise en œuvre d'une poche souple dans le réservoir du flacon permet ainsi la délivrance immédiate du produit, et permet en outre la délivrance totale ou quasi-totale du produit contenu dans le flacon.

Lorsque l'utilisateur applique une pression sur la paroi déformable, l'air présent entre la paroi déformable du réservoir et la poche souple est empêché de sortir de cet espace (que ce soit par un moyen qui empêche automatiquement cette sortie de l'air ou par une action de l'utilisateur), de sorte que la poche souple est elle-même mise en pression, provoquant l'expulsion du produit au travers de l'embout goutte à goutte.

En outre, afin de rendre le flacon adapté à la distribution d'un produit ophtalmique visqueux sans conservateur, les surfaces du flacon susceptibles d'être au contact du produit et qui sont situées en aval de la valve unidirectionnelle portent un traitement antibactérien et/ou un traitement qui en diminue la mouillabilité.

Dans l'ensemble du présent document, les termes « amont » et « aval » s'entendent en considérant le sens d'écoulement du produit ophtalmique visqueux lors de sa délivrance, c'est-à-dire depuis le réservoir jusqu'à sa sortie de l'embout du flacon.

Un traitement bactéricide des surfaces internes situées en aval de la valve unidirectionnelle et/ou un traitement qui réduit la mouillabilité de ces surfaces permet, en combinaison avec les autres caractéristiques du flacon, de conditionner le produit ophtalmique sans conservateur. En effet, suite à la délivrance, tout retour de produit vers le réservoir est empêché par la valve unidirectionnelle. Cela permet de maintenir la stérilité du produit présent dans le réservoir. Un reliquat de produit peut néanmoins rester entre la valve unidirectionnelle et l'orifice de distribution du flacon. Le traitement antibactérien évite tout développement bactérien dans ce reliquat de produit, qui, même s'il s'agit d'une très faible quantité, sera expulsé du flacon lors de la prochaine distribution. Par ailleurs, le traitement évite tout développement bactérien sur les surface internes au flacon qui portent le traitement.

En complément ou en alternative, un traitement permettant de limiter la mouillabilité permet de limiter l'éventuel reliquat de produit ophtalmique, après une distribution, en aval de la valve unidirectionnelle du flacon. La présence d'un tel reliquat est d'autant plus probable, et le reliquat d'autant plus important, que le produit ophtalmique est visqueux. Il est rappelé que la mouillabilité peut être définie comme l'aptitude d'une surface à être mouillée par un liquide. Elle peut se mesurer par l'angle de contact d'une goutte d'un liquide posé sur la surface considérée. Une faible mouillabilité correspond à une situation dans laquelle le produit a tendance à s'écouler facilement sur la surface, et ne tend pas à y adhérer. Un tel revêtement permet donc de limiter le risque de développement bactérien en aval de la valve unidirectionnelle, et aussi de garantir une meilleure reproductibilité des doses délivrées par le flacon, malgré le caractère visqueux du produit.

Le flacon contenant un produit ophtalmique visqueux stérile et sans conservateur répond ainsi, par une combinaison de caractéristiques appropriée, à la double problématique de la conservation de la stérilité du produit et de la délivrance aisée.

La poche souple peut être formée par délamination d'une couche interne du réservoir. En alternative à une poche souple présente dès la fabrication dans le réservoir du flacon, une poche souple obtenue par délamination de la paroi du réservoir est une solution simple, maitrisée industriellement, et peu coûteuse, pour obtenir la fonction recherchée dans l'invention.

Alternativement, la poche souple peut être une poche souple rétractable, qui est rapportée. L'emploi d'une telle poche souple rétractable permet notamment de sélectionner une poche ayant une contenance nominale correspondant précisément au volume initial de produit ophtalmique visqueux à conditionner, tout en employant une même paroi déformable pour différents volumes de produit à conditionner.

Le moyen d'entrée d'air peut être formé d'un trou dans la paroi du réservoir adapté à être obturé par un doigt de l'utilisateur.

Il s'agit d'une solution particulièrement simple pour empêcher la sortie de l'air contenu entre la paroi du réservoir et la poche souple lors de la distribution de produit vers l'extérieur du réservoir, c'est-à-dire lorsqu'une pression est appliquée sur le réservoir. Un positionnement adapté du trou ménagé dans la paroi rend l'utilisation du flacon parfaitement intuitive. Le trou peut ainsi être avantageusement positionné en une zone du réservoir où un doigt de l'utilisateur vient naturellement se poser lorsqu'il tient le flacon en vue de distribuer du produit contenu par le flacon. Le trou peut ainsi être positionné sur une paroi latérale du réservoir, notamment à proximité du col du réservoir, ou, alternativement, sur le fond du réservoir.

Alternativement, le moyen d'entrée d'air peut comporter un clapet unidirectionnel, configuré pour permettre l'entrée d'air entre la paroi du réservoir et la poche souple et pour empêcher la sortie vers l'extérieur du réservoir de l'air présent entre la paroi du réservoir et la poche souple.

Selon cette configuration, la sortie d'air vers l'extérieur du réservoir est empêchée automatiquement, ce qui rend l'utilisation du réservoir extrêmement simple, car l'utilisateur n'a aucune action particulière à faire (aussi simple soit-elle) comparativement à un flacon classique à réservoir déformable.

Le traitement antibactérien peut être à base d'ions argent ou d'ions zinc.

Le traitement antibactérien peut comporter des ions argent ou des ions zinc inclus dans la masse des éléments formants lesdites surfaces internes.

Alternativement ou en complément, le traitement bactéricide peut comporter un revêtement de surface bactéricide.

Ce type de traitement antibactérien se révèle tout à fait adapté aux produits ophtalmiques, destinés à être appliqués sur l'œil d'un patient.

Bien évidemment, le traitement antibactérien peut également avoir un effet antifongique.

Le traitement qui diminue la mouillabilité des surfaces internes situées entre la valve unidirectionnelle et l'orifice de distribution peut être un traitement de type traitement plasma fluoré ou siliconé.

Le traitement qui diminue la mouillabilité des surfaces internes situées entre la valve unidirectionnelle et l'orifice de distribution peut être une texturation de surface par laser.

Le flacon contenant un produit ophtalmique visqueux peut comporter un capuchon amovible, rapporté sur la tête de distribution.

Le capuchon assure une fermeture étanche à l'air du flacon. Il participe ainsi à garantir le maintien de la stérilité du produit présent dans le réservoir. Il garantit aussi la protection de l'embout de distribution, notamment contre d'éventuelles contaminants (poussières, bactéries, etc.) entre deux distributions de doses du produit ophtalmique. L'éventuel reliquat de produit en aval de la valve unidirectionnelle du flacon est ainsi protégé.

La valve unidirectionnelle peut comporter un élément déformable sous l'effet d'une surpression à l'intérieur du réservoir du flacon, ou comporter un clapet à bille.

Le produit ophtalmique visqueux contenu dans le flacon peut comporter un ou plusieurs actifs en quantité thérapeutiquement efficace choisi dans le groupe des anti-glaucomateux, des anti-inflammatoires, des immunosuppresseurs, des antiviraux, des antibactériens des antifongiques ou des anti-oxydants. Le produit ophtalmique visqueux contenu dans le flacon peut comporter un ou plusieurs actifs en quantité thérapeutiquement efficace contre la sècheresse oculaire tels que les dérivés acide polyacrylique, les carbomères, le tréhalose, l'acide hyaluronique ou ses sels, la povidone, l'alcool polyvinylique, les dérivés de cellulose, les dérivés de chitosan, les phospholipides, les triglycérides.

Par actif on entend un composé administré à un sujet présentant les symptômes (signes) d'une pathologie dans le but de réduire ou de supprimer ces symptômes. L'expression « quantité thérapeutiquement efficace » se réfère à une quantité d'actif qui est suffisante ou efficace pour prévenir ou traiter une maladie ou un trouble y compris le soulagement des symptômes de cette maladie ou de ce trouble.

D'autres particularités et avantages de l'invention apparaîtront encore dans la description ci-après.

Aux dessins annexés, donnés à titre d'exemples non limitatifs :
- la figure 1 représente, selon une vue en coupe, un flacon conforme à un mode de réalisation de l'invention ;
- la figure 2 représente, selon une vue en coupe, le flacon de la figure 1 lors de la délivrance du produit qu'il contient ;
- la figure 3 représente, selon une vue en coupe, un flacon conforme à un mode de réalisation de l'invention, illustrant plusieurs aspects pouvant être mis en œuvre dans un tel flacon ;
- la figure 4 représente, selon une vue en coupe, un flacon conforme à un autre mode de réalisation de l'invention, illustrant plusieurs aspects pouvant être mis en œuvre dans un tel flacon ;
- la figure 5 représente, selon une vue en coupe, un flacon conforme à un autre mode de réalisation de l'invention ;
- la figure 6 représente, selon une vue en coupe partielle, un flacon conforme à encore un autre mode de réalisation de l'invention.

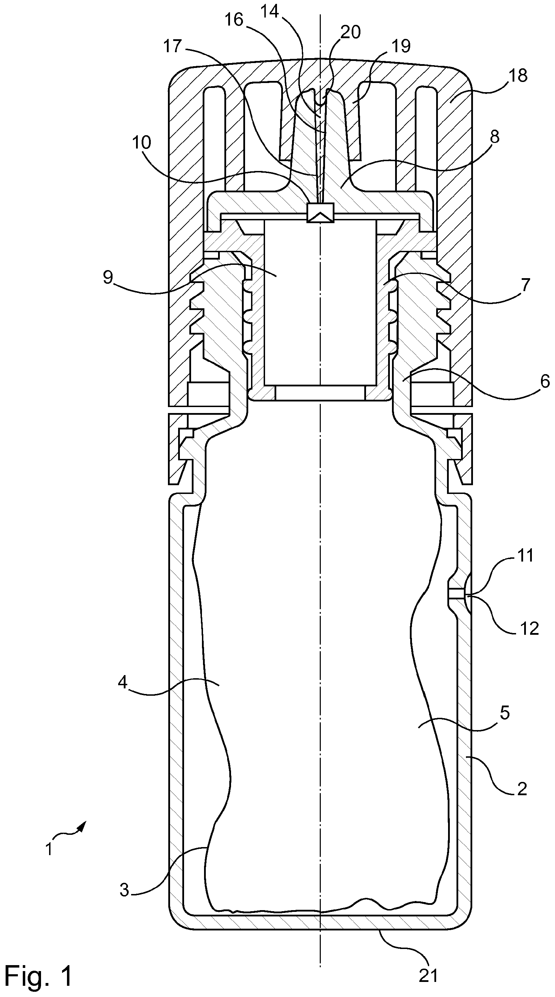

La figure 1 représente un flacon contenant un produit ophtalmique visqueux selon un premier mode de réalisation de l'invention.

Le réservoir 1 comporte une paroi 2 déformable, et qui forme un volume interne du réservoir 1. La paroi 2 est en particulier configurée pour pouvoir être déformée sous une pression exercée par la main d'un utilisateur lorsque celui-ci souhaite procéder à la délivrance d'une ou plusieurs gouttes de produit.

Dans l'exemple représenté, la paroi 2 est une paroi périphérique cylindrique. La paroi 2 est élastiquement déformable, c'est-à-dire qu'elle tend spontanément à reprendre sa forme initiale après qu'une pression a cessé d'être exercée sur elle.

Le réservoir comporte, dans son volume interne, une poche souple 3. La poche souple 3 peut être une poche rapportée, formée en un matériau plastique souple, en silicone, etc. Alternativement, la poche souple 3 est formée par délamination de la surface interne de la paroi 2, c'est-à-dire que la poche souple 3 se détache de la paroi 2 lors des premières délivrances de produit. La poche souple forme un volume de réception 4 dans lequel est contenu le produit ophtalmique visqueux.

La poche souple 3 du réservoir 1 contient un produit ophtalmique visqueux 5, qui est stérile et sans conservateur.

La poche souple doit donc présenter des caractéristiques de perméabilité suffisante pour assurer une bonne conservation du produit qu'elle contient sur une longue période, typiquement de plusieurs semaines ou plusieurs mois.

Dans l'exemple représenté à la figure 1, la poche souple se maintient au contact de la paroi 2 du réservoir au moins à proximité d'un col 6 du réservoir.

Une tête de distribution est rapportée au flacon. Dans l'exemple de mode de réalisation ici représenté, la tête de distribution du produit ophtalmique visqueux en goutte à goutte comprend un insert 7, disposé à l'intérieur du col 6 du flacon. Un embout 8 (ou buse) de distribution goutte à goutte est fixé au (ou formé par) l'insert 7. L'insert 7 est fixé rigidement et de manière étanche dans le col 6. L'insert 7 peut par exemple être inséré en force dans le col 6.

L'insert 7 est dit évidé, en ce qu'il ménage un espace 9 permettant le passage du produit ophtalmique visqueux de la poche souple 3 vers l'embout 8.

Le flacon comporte également une valve unidirectionnelle 10. La valve unidirectionnelle est configurée, notamment orientée, de sorte à se laisser traverser par le produit ophtalmique visqueux 5 sous l'effet d'une surpression à l'intérieur du réservoir 1 comparativement à l'extérieur (à la pression atmosphérique).

Dans l'exemple représenté, cette valve unidirectionnelle 10 est interposée dans le passage ménagé pour le produit, à l'entrée de l'embout 8 de distribution.

La valve unidirectionnelle ici représentée à titre d'exemple comporte une lèvre déformable sous l'effet d'une surpression dans le réservoir 1 du flacon. Au repos, en l'absence de différence de pression de part et d'autre de la valve unidirectionnelle 10 cette dernière est hermétiquement close, et toute dépression dans le réservoir tend à fermer encore plus fortement la valve unidirectionnelle.

Le flacon comporte en outre, un moyen d'entrée d'air 11. Le moyen d'entrée d'air 11 permet à l'air de traverser la paroi 2 du flacon 1, de sorte à prendre place entre ladite paroi 2 et la poche souple 3. Dans l'exemple ici représenté, le moyen d'entrée d'air 11 est formé d'un trou 12, par exemple un trou rond de quelques millimètres de diamètre. Le trou 12 peut être formé dans un léger renfoncement de la paroi 2.

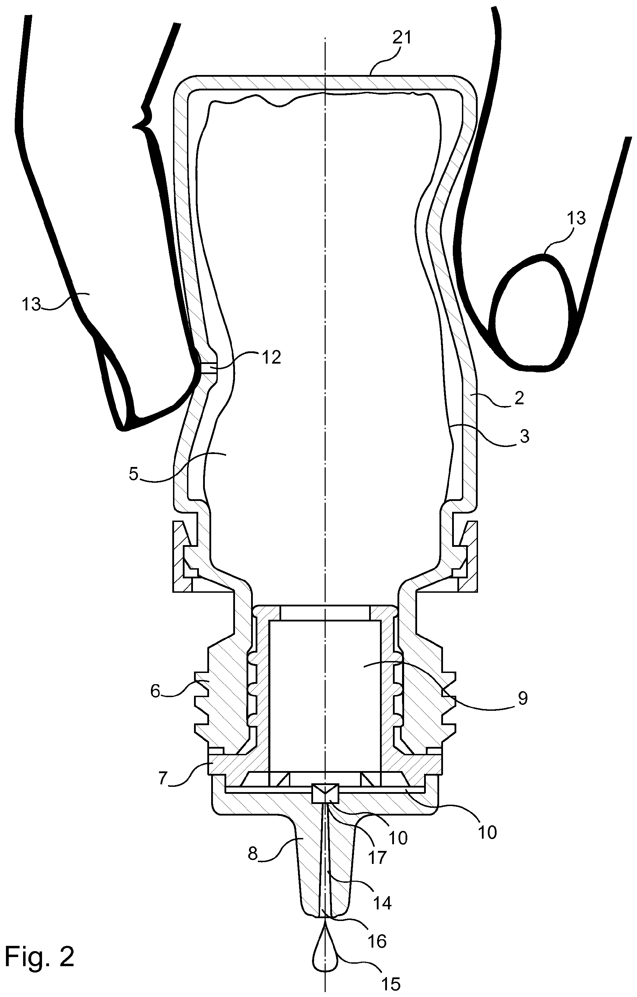

La figure 2 représente le flacon de la figure 1 lors de la délivrance du produit qu'il contient. Pour entrainer la délivrance d'une ou plusieurs gouttes de produit ophtalmique visqueux, un utilisateur presse le flacon par exemple entre deux doigts 13, déformant ainsi la paroi 2, et réduisant son volume interne. Lors de la compression manuelle du flacon, l'un des doigts 13 est placé de sorte à obturer le trou 12, ce qui empêche l'air présent entre la paroi 2 et la poche souple 3 de s'évacuer. Il en résulte une augmentation de la pression dans le réservoir, tant pour ce qui concerne l'air présent entre la paroi 2 et la poche souple 3 que pour le produit ophtalmique visqueux, la pression étant transmise par la poche souple 3.

La différence de pression entre le volume interne du réservoir 1 et l'air ambiant entraine un flux de produit ophtalmique visqueux contenu dans le réservoir au travers de la valve unidirectionnelle 10. Le produit ophtalmique visqueux la traverse librement sous l'effet de la pression générée par la force appliquée sur les parois du flacon par l'utilisateur, du fait de l'orientation de la valve unidirectionnelle 10.

Le produit ophtalmique visqueux s'évacue ensuite par l'embout 8, par exemple via un canal 14 de faible section formé dans l'embout 8 et permettant la formation de gouttes 15 régulières.

Lorsque l'utilisateur relâche la pression qu'il exerce sur le réservoir 1, son doigt 13 cesse d'obturer de manière étanche le trou 12. L'air est ainsi libre d'entrer dans l'espace formé entre la paroi 2 et la poche souple 3. Dans le même temps, la valve unidirectionnelle 10 s'oppose à la ré-aspiration d'air dans la poche souple 3 via l'embout 8. Il en résulte que le volume de produit ophtalmique visqueux expulsé sera totalement compensé par l'entrée d'un volume d'air correspondant dans le réservoir 1 entre la paroi 2 et la poche souple 3.

Néanmoins, et en particulier dans la mesure où le produit délivré est visqueux, un reliquat de produit peut adhérer aux surfaces internes situées en aval de la valve unidirectionnelle 10. Ces surfaces comportent les surfaces 16 du canal 14, ainsi que la surface extérieure 17 de la valve unidirectionnelle 10. La surface dite extérieure de la valve unidirectionnelle 10 est sa surface orientée vers l'orifice de distribution du flacon, c'est-à-dire la surface située à la base du canal 14. Plus généralement, ces surfaces correspondent aux surfaces susceptibles d'être au contact du produit ophtalmique visqueux lors de sa distribution, et pouvant par ailleurs être au contact de l'air extérieur au flacon, au moins dans une situation d'utilisation du flacon (typiquement lors de la distribution du produit ophtalmique visqueux).

La valve unidirectionnelle 10 peut avantageusement être positionnée à l'extrémité de l'embout 8, minimisant ainsi la quantité de liquide résiduel en aval de la valve unidirectionnelle. Néanmoins, ces surface internes en aval de la valve unidirectionnelle ne peuvent pas être totalement éliminées car elles participent à la formation de gouttes régulières.

Ces surfaces internes situées en aval de la valve unidirectionnelle 10 sont traitées, et portent un traitement antibactérien et/ou un traitement qui diminue leur mouillabilité. Ces deux types de traitement peuvent, alternativement ou en combinaison, rendre le flacon adapté à la délivrance d'un produit ophtalmique visqueux stérile sans conservateur.

En effet, la zone dans laquelle ces surfaces sont présentes et où un reliquat de produit est susceptible de rester après la distribution d'une dose de produit ophtalmique visqueux n'est pas protégée, au moins lors de la distribution du produit, contre l'arrivée de contaminants (poussières, bactéries) extérieurs au flacon. Ainsi, un traitement antibactérien, par exemple un traitement de surface au niveau des surfaces 16 du canal 14 et de la surface extérieure 17 de la valve unidirectionnelle 10 et/ou dans la masse de l'embout 8 et/ou de la valve unidirectionnelle 10, permet de garantir l'absence de prolifération bactérienne sur ces surfaces, ainsi que le cas échéant dans le reliquat de produit qui serait au contact de ces surfaces.

Un traitement qui limite la mouillabilité de ces surfaces a pour effet de limiter ou d'éviter le reliquat de produit en aval de la valve unidirectionnelle 10. La question d'une prolifération bactérienne excessive dans un reliquat de produit est ainsi évitée. Idéalement, le traitement antibactérien et le traitement qui limite la mouillabilité sont combinés, de sorte que la présence de reliquat est limitée par le traitement qui limite la mouillabilité tandis que tout développement bactérien ou fongique est évité sur les surfaces traitées.

Le flacon proposé selon la présente invention comporte ainsi une combinaison de caractéristiques qui le rendent adapté au conditionnement et à la délivrance de multiples doses du produit ophtalmique visqueux stérile et sans conservateur qu'il contient.

De nombreux autres aspects pouvant être mis en œuvre sur un tel flacon sont illustrés aux figures suivantes.

Afin de garantir plus encore l'absence d'aspiration d'air dans la poche souple 3 et plus généralement au contact du produit ophtalmique visqueux en amont de la valve unidirectionnelle 10, divers moyens peuvent être employés en alternatives ou en compléments les uns des autres. En premier lieu, un capuchon 18 amovible d'obturation de l'embout 8 peut être utilisé. Le capuchon 18 peut être en particulier adapté à se visser autour du col 6 du flacon. Le capuchon assure une étanchéité à l'air grâce à son mode de fixation sur le col 6 du flacon, et/ou grâce à des moyens complémentaires. Notamment, un cylindre de réception 19 de l'embout 8 peut être formé dans le capuchon 18, ainsi qu'un pion 20 d'obturation du canal 14. Ainsi, une fois le flacon fermé, entre deux distributions de produit, aucune entrée d'air via l'embout 8 de distribution ne peut se produire. Par ailleurs, le capuchon 18 assure également une protection hermétique de la partie interne du flacon située en aval de la valve unidirectionnelle 10, et en particulier une protection du canal 14.

Dans l'exemple de mode de réalisation représenté aux figures 1 et 2, le moyen d'entrée d'air 11 est formé d'un trou 12 pouvant être obstrué facilement par l'utilisateur. Le positionnement du trou 12, s'il doit être naturel pour l'utilisateur, peut cependant être adapté selon le flacon. Il peut être en partie haute de la paroi 2, en son milieu (selon la direction générale d'extension du flacon), ou encore sur le fond 21 du flacon.

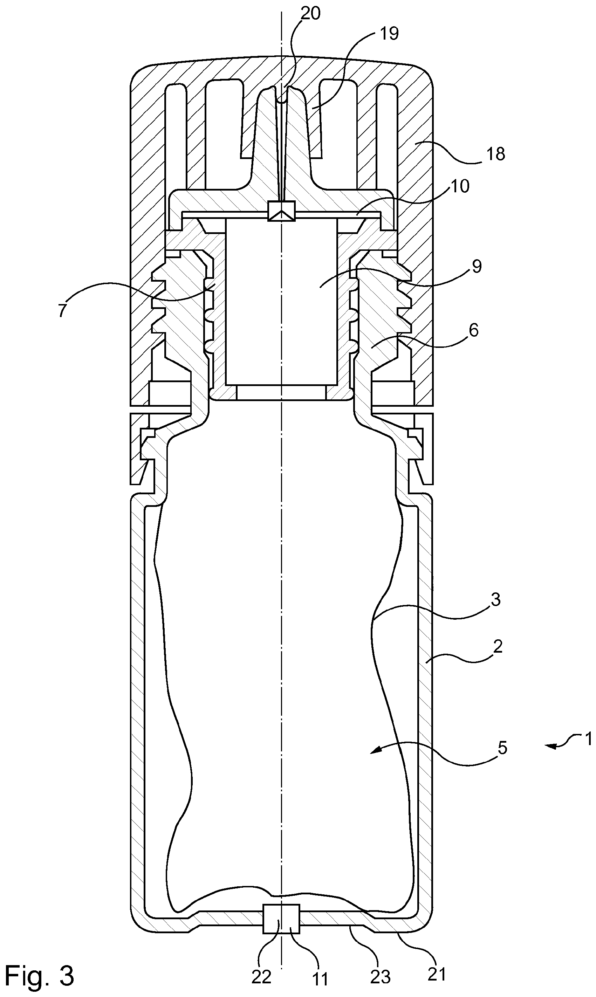

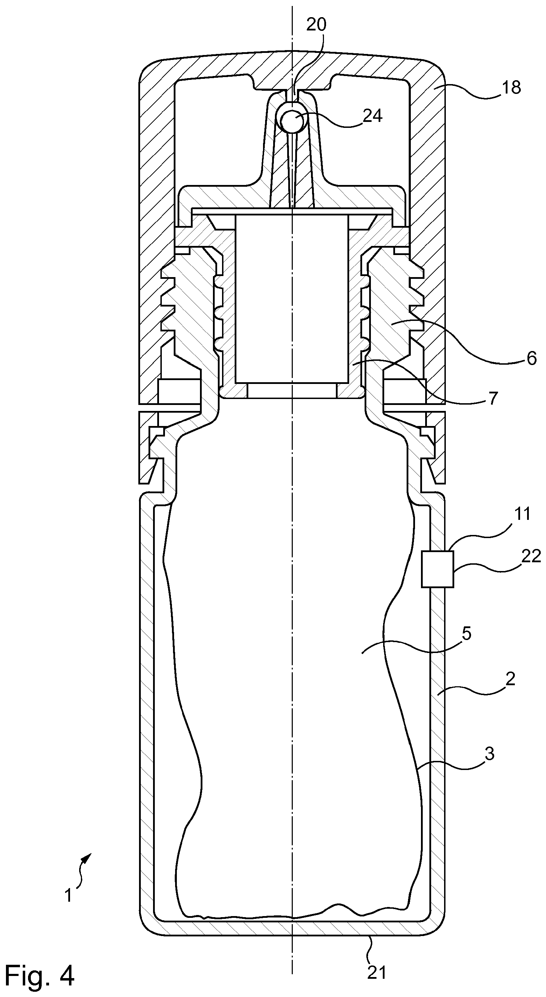

Comme illustré à la figure 3 et à la figure 4, le trou 12 peut être remplacé par un clapet unidirectionnel 22, laissant entrer l'air dans l'espace entre la paroi 2 et la poche souple 3 mais l'empêchant de ressortir de cet espace. Le clapet unidirectionnel 22 peut être une valve à lèvres souples, un clapet à bille, etc. Il peut être situé sur la paroi 2 comme représenté à la figure 4 ou sur le fond 21 du flacon comme représenté à la figure 3. Dans le mode de réalisation de la figure 3, le clapet unidirectionnel 22 est placé dans un renfoncement 23 formé dans le fond 1 du flacon.

Dans l'exemple représenté à la figure 4, la valve unidirectionnelle qui permet la distribution du produit et empêche le retour d'air dans le réservoir est un clapet à bille 24. Le clapet à bille 24 offre la même fonctionnalité que la valve unidirectionnelle à lèvre déformable décrite précédemment. La bille porte alors avantageusement (ainsi que les surfaces sur logement où elle se situe) le traitement antibactérien et/ou limitant la mouillabilité.

Dans l'exemple représenté à la figure 4, le clapet à bille est formé à proximité de l'orifice de distribution du produit ophtalmique visqueux, c'est-à-dire de la sortie du canal 14. Cela limite les surfaces internes situées en aval de la valve unidirectionnelle. Cette disposition peut bien évidemment être adoptée avec toute technologie de valve unidirectionnelle. Elle peut cependant limiter la régularité des gouttes issues du flacon.

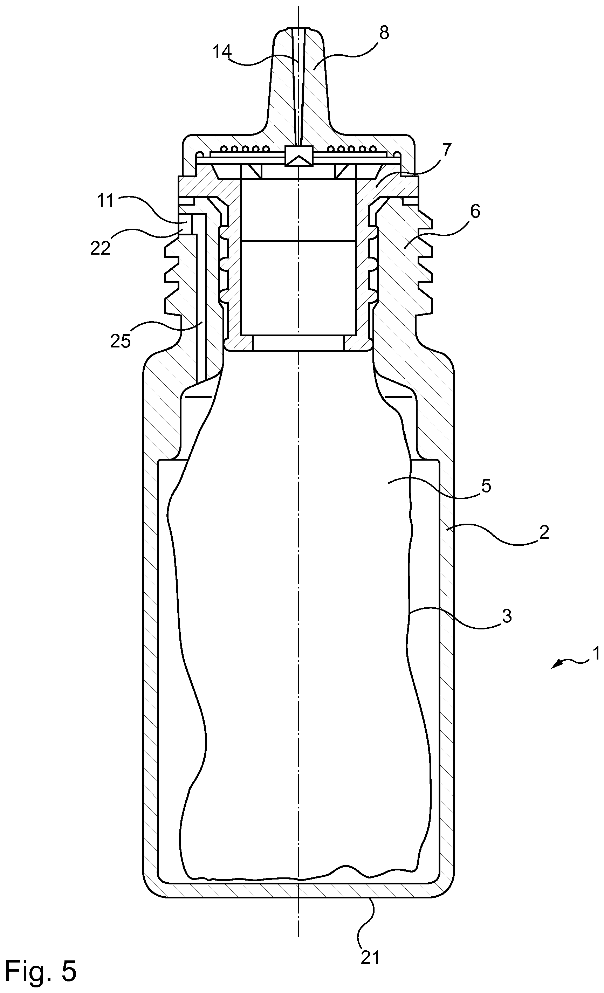

La figure 5 présente un mode de réalisation dans lequel le moyen d'entrée d'air 11 est intégré en partie haute du flacon, à savoir dans l'exemple représenté dans son col 6. Un conduit 25 permet de guider l'air entrant par le moyen d'entrée d'air vers l'espace situé entre la paroi 2 et la poche souple 3. Un clapet unidirectionnel 22, formant moyen d'entrée d'air 11, est disposé à l'embouchure du conduit 25. Ce mode de réalisation permet une intégration discrète du moyen d'entrée d'air et propose un positionnement pour ce moyen qui évite tout risque d'obturation involontaire lors de la distribution du produit contenu dans le flacon.

Alternativement au clapet unidirectionnel, le moyen d'entrée d'air 11 peut être équipé (dans tout mode de réalisation de l'invention) d'un élément perméable à l'air mais adapté à générer une perte de charge importante (de sorte que la diffusion de l'air au travers de cet élément ne peut se faire que lentement). Un fin opercule perméable à l'air, par exemple en silicone, peut être utilisé pour former cet élément.

Ainsi, lors de la délivrance du produit, lorsque l'utilisateur presse le réservoir 1, un différentiel de pression important est créé mais la résistance contre l'expulsion du produit par la valve unidirectionnelle 10 et l'embout 8 est moindre que la résistance au passage de l'air générée par l'opercule, et le temps nécessaire à la distribution est trop court pour permettre un passage significatif de l'air au travers de l'opercule.

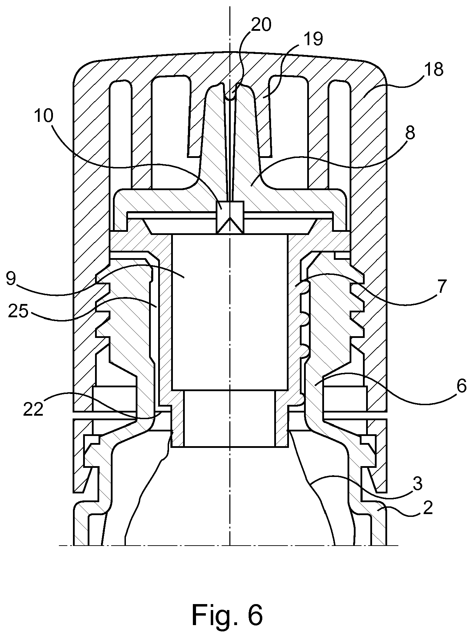

Lorsque la pression sur le réservoir est relâchée, l'entrée d'air par l'embout 8 étant empêchée par la valve unidirectionnelle 10, la différence de pression entre le volume intérieur du réservoir et l'extérieur entraine le passage progressif d'air au travers de l'opercule, jusqu'à ce que les pressions soient équilibrées. La compensation du volume de produit distribué par de l'air entre la paroi 2 et la poche souple 3 est ainsi réalisée. La figure 6 présente un autre mode de réalisation de l'invention, dans lequel la poche souple 3 est une poche souple rapportée et le moyen d'entrée d'air comporte un élément formant clapet unidirectionnel 22 au niveau de l'interface entre la poche souple et l'élément du flacon auquel la poche souple est liée.

Dans l'exemple ici représenté la poche souple 3 est liée à l'insert 7 (cette configuration étant d'ailleurs applicable à tout mode de réalisation comportant une poche rapportée). L'élément formant clapet unidirectionnel est ouvert et permet le passage de l'air en l'absence de différence de pression entre l'extérieur du flacon et le volume interne du réservoir, ou est configuré pour s'ouvrir dès qu'une faible dépression est générée dans le volume interne du réservoir. Par contre, dès que le volume interne est en surpression comparativement à la pression extérieure au flacon (pression atmosphérique), l'élément formant clapet unidirectionnel 22 se ferme et empêche l'air de s'échapper de l'espace situé entre la paroi 2 et la poche souple 3.

En particulier, l'élément formant clapet unidirectionnel 22 peut être formé d'une fine collerette ou d'un joint plat pouvant soit être plaqué sur la sortie d'un conduit 25 reliant l'extérieur du flacon et le volume interne du réservoir, ce qui obture le conduit 25, soit au contraire se décoller ou se déformer pour libérer le conduit 25, selon le différentiel de pression entre l'intérieur et l'extérieur du flacon. Un opercule perméable à l'air tel que décrit ci-dessus peut aussi être utilisé dans une variante de ce mode de réalisation.

Le mode de réalisation de la figure 6 offre les avantages de celui de la figure 5, et est simple à mettre en œuvre.

Bien évidemment, les différents aspects présentés ci-dessus en référence aux figures données à titre d'exemple peuvent être combinés pour former d'autres modes de réalisation de l'invention.

Le flacon ainsi développé permet le conditionnement « multidoses » et la délivrance du produit ophtalmique visqueux stérile sans conservateur qu'il contient.

Le volume de la poche s'adaptant à celui du produit ophtalmique visqueux, tout le produit peut être distribué, sans difficulté, y compris les dernières doses présentes dans le flacon, sans déformation résiduelle de la paroi du réservoir.

## Revendications

1. Flacon contenant un produit ophtalmique pouvant être administré sous forme de gouttes,
le flacon comportant un réservoir (1) comportant une paroi (2) définissant un volume interne et adaptée à se déformer sous l'effet d'une pression exercée sur le réservoir (1) par un utilisateur du flacon et à reprendre spontanément sa forme d'origine après relâchement de ladite pression,
le réservoir (1) comportant en outre une poche souple (3) dans le volume interne défini par la paroi (2) et qui contient le produit ophtalmique,
le flacon comportant :
- un moyen d'entrée d'air (11) entre la paroi (2) du réservoir (1) et la poche souple (3),
- une tête rapportée sur un col (6) du réservoir (1) et comportant un embout (8) goutte à goutte comportant un orifice de distribution, et
- une valve unidirectionnelle (10) interposée entre la poche souple (3) et l'orifice de distribution, la valve unidirectionnelle étant configurée pour empêcher l'entrée d'air provenant de l'extérieur du flacon dans la poche souple (3), de sorte que suite à une distribution de produit ophtalmique, le volume de produit ophtalmique distribué est compensé par l'entrée d'un volume d'air correspondant entre la paroi (2) du réservoir (1) et la poche souple (3) via le moyen d'entrée d'air (11),
**caractérisé en ce que** le produit ophtalmique est un produit ophtalmique visqueux (5), c'est-à-dire ayant une viscosité supérieure ou égale à 200 cP et est stérile et sans conservateur,
et **en ce que**, le flacon comportant des surfaces internes situées entre la valve unidirectionnelle (10) et l'orifice de distribution, la totalité desdites surfaces internes portent un traitement antibactérien et/ou un traitement qui en diminue la mouillabilité.

2. Flacon contenant un produit ophtalmique visqueux selon la revendication 1, dans lequel la poche souple (3) est formée par délamination d'une couche interne du réservoir (1).

3. Flacon contenant un produit ophtalmique visqueux selon la revendication 1, dans lequel la poche souple (3) est une poche souple rétractable.

4. Flacon contenant un produit ophtalmique visqueux selon l'une des revendications 1 à 3, dans lequel le moyen d'entrée d'air (11) est formé d'un trou (12) dans la paroi (2) du réservoir (1) adapté à être obturé par un doigt (13) de l'utilisateur.

5. Flacon contenant un produit ophtalmique visqueux selon l'une des revendications 1 à 3, dans lequel le moyen d'entrée d'air (11) comporte un clapet unidirectionnel (22), configuré pour permettre l'entrée d'air entre la paroi (2) du réservoir (1) et la poche souple (3) et pour empêcher la sortie vers l'extérieur du réservoir (1) de l'air présent entre la paroi (2) du réservoir (1) et la poche souple (3).

6. Flacon contenant un produit ophtalmique visqueux selon l'une des revendications précédentes, dans lequel le traitement antibactérien est à base d'ions argent ou d'ion zinc.

7. Flacon contenant un produit ophtalmique visqueux selon la revendication 6, dans lequel le traitement antibactérien comporte des ions argent ou des ions zinc inclus dans la masse des éléments formants lesdites surfaces internes situées entre la valve unidirectionnelle et l'orifice de distribution.

8. Flacon contenant un produit ophtalmique visqueux selon l'une des revendications 1 à 7, dans lequel le traitement antibactérien comporte un revêtement de surface bactéricide.

9. Flacon contenant un produit ophtalmique visqueux selon l'une des revendications précédentes, dans lequel une surface extérieure (17) de la valve unidirectionnelle (10) orientée vers l'orifice de distribution, porte également le traitement antibactérien.

10. Flacon contenant un produit ophtalmique visqueux selon l'une des revendications précédentes dans lequel le traitement qui diminue la mouillabilité des surfaces internes situées entre la valve unidirectionnelle (10) et l'orifice de distribution est un traitement de type traitement plasma fluoré ou siliconé.

11. Flacon contenant un produit ophtalmique visqueux selon l'une des revendications 1 à 9 dans lequel le traitement qui diminue la mouillabilité des surfaces internes situées entre la valve unidirectionnelle (10) et l'orifice de distribution est une texturation de surface par laser.

12. Flacon contenant un produit ophtalmique visqueux selon l'une des revendications précédentes comportant un capuchon (18) amovible, rapporté sur la tête de distribution.

13. Flacon contenant un produit ophtalmique visqueux selon l'une des revendications précédentes dans lequel la valve unidirectionnelle (10) comporte un élément déformable sous l'effet d'une surpression à l'intérieur du réservoir du flacon, ou comporte un clapet à bille (24).

14. Flacon contenant un produit ophtalmique visqueux selon l'une des revendications précédentes, dans lequel ledit produit ophtalmique visqueux (5) comporte un ou plusieurs actifs en quantité thérapeutiquement efficace choisi dans le groupe des anti-glaucomateux, des anti-inflammatoires, des immunosuppresseurs, des antiviraux, des antibactériens des antifongiques ou des anti-oxydants, et/ou un ou plusieurs actifs en quantité thérapeutiquement efficace contre la sècheresse oculaire tels que les dérivés acide polyacrylique, les carbomères, le tréhalose, l'acide hyaluronique ou ses sels, la povidone, l'alcool polyvinylique, les dérivés de cellulose, les dérivés de chitosan, les phospholipides, les triglycérides.

## Patentansprüche

1. Flasche, die ein Augenprodukt enthält, das in Tropfenform verabreicht werden kann,
wobei die Flasche einen Behälter (1) aufweist, der eine Wand (2) aufweist, die ein Innenvolumen definiert und dazu ausgelegt ist, sich unter der Wirkung eines Drucks, der von einem Benutzer der Flasche auf den Behälter (1) ausgeübt wird, zu verformen und nach dem Loslassen des Drucks spontan seine ursprüngliche Form wieder einzunehmen;
wobei der Behälter (1) weiter einen flexiblen Beutel (3) in dem durch die Wand (2) begrenzten Innenvolumen aufweist, der das Augenprodukt enthält,
wobei die Flasche Folgendes aufweist:
- ein Lufteinlassmittel (11) zwischen der Wand (2) des Behälters (1) und dem flexiblen Beutel (3),
- einen an einem Hals (6) des Behälters (1) angebrachten Kopf, der eine Tropfspitze (8) aufweist, die eine Abgabeöffnung aufweist, und
- ein Einwegventil (10), das zwischen dem flexiblen Beutel (3) und der Abgabeöffnung angeordnet ist, wobei das Einwegventil so ausgebildet ist, dass es das Einlassen von Luft von außerhalb der Flasche in den flexiblen Beutel (3) verhindert, so dass infolge einer Verteilung des Augenprodukts das abgegebene Volumen des Augenprodukts durch das Einlassen eines entsprechenden Luftvolumens zwischen der Wand (2) des Behälters (1) und dem flexiblen Beutel (3) über das Lufteinlassmittel (11) ausgeglichen wird,
**dadurch gekennzeichnet, dass** das Augenprodukt ein viskoses Augenprodukt (5) ist, d. h., eine Viskosität von mindestens 200 cP aufweist und steril und konservierungsmittelfrei ist,
und dadurch, dass die Flasche Innenflächen aufweist, die sich zwischen dem Einwegventil (10) und der Abgabeöffnung befinden, wobei alle diese Innenflächen eine antibakterielle Behandlung und/oder eine Behandlung tragen, die ihre Benetzbarkeit verringert.

2. Flasche mit einem viskosen Augenprodukt nach Anspruch 1, wobei der flexible Beutel (3) durch Delaminieren einer Innenschicht des Behälters (1) gebildet wird.

3. Flasche nach Anspruch 1, wobei der flexible Beutel (3) ein schrumpfbarer flexibler Beutel ist.

4. Flasche mit einem viskosen Augenprodukt nach einem der Ansprüche 1 bis 3, wobei das Lufteinlassmittel (11) durch ein Loch (12) in der Wand (2) des Behälters (1) gebildet ist, das mit einem Finger (13) des Benutzers verschlossen werden kann.

5. Flasche mit einem viskosen Augenprodukt nach einem der Ansprüche 1 bis 3, wobei das Lufteinlassmittel (11) ein Einwegventil (22) aufweist, das so ausgebildet ist, dass es das Einlassen von Luft zwischen der Wand (2) des Behälters (1) und dem flexiblen Beutel (3) ermöglicht und das Ablassen der zwischen der Wand (2) des Behälters (1) und dem flexiblen Beutel (3) vorhandenen Luft nach außen aus dem Behälter (1) verhindert.

6. Flasche mit einem viskosen Augenprodukt nach einem der vorhergehenden Ansprüche, wobei die antibakterielle Behandlung auf Silberionen- oder Zinkionenbasis erfolgt.

7. Flasche mit einem viskosen Augenprodukt nach Anspruch 6, wobei die antibakterielle Behandlung Silberionen oder Zinkionen aufweist, die in der Masse der Elemente eingeschlossen sind, die die Innenflächen bilden, die sich zwischen dem Einwegventil und der Abgabeöffnung befinden.

8. Flasche mit einem viskosen Augenprodukt nach einem der Ansprüche 1 bis 7, wobei die antibakterielle Behandlung eine bakterizide Oberflächenbeschichtung aufweist.

9. Flasche mit einem viskosen Augenprodukt nach einem der vorhergehenden Ansprüche, wobei eine zur Abgabeöffnung gerichtete Außenfläche (17) des Einwegventils (10) auch die antibakterielle Behandlung trägt.

10. Flasche mit einem viskosen Augenprodukt nach einem der vorhergehenden Ansprüche, wobei die Behandlung, die die Benetzbarkeit der Innenflächen zwischen dem Einwegventil (10) und der Abgabeöffnung verringert, eine Behandlung des Typs Fluor- oder Silikonplasmabehandlung ist.

11. Flasche mit einem viskosen Augenprodukt nach einem der Ansprüche 1 bis 9, wobei die Behandlung, die die Benetzbarkeit der Innenflächen zwischen dem Einwegventil (10) und der Abgabeöffnung verringert, eine Oberflächentexturierung mit Laser ist.

12. Flasche mit einem viskosen Augenprodukt nach einem der vorhergehenden Ansprüche, die eine abnehmbare Kappe (18) aufweist, die auf den Abgabekopf aufgebracht ist.

13. Flasche mit einem viskosen Augenprodukt nach einem der vorhergehenden Ansprüche, wobei das Einwegventil (10) ein Element aufweist, das sich unter der Wirkung eines Überdrucks im Inneren des Behälters der Flasche verformt, oder ein Kugelventil (24) aufweist.

14. Flasche mit einem viskosen Augenprodukt nach einem der vorstehenden Ansprüche, wobei das viskose Augenprodukt (5) einen oder mehrere Wirkstoffe in therapeutisch wirksamer Menge, der aus der Gruppe ausgewählt ist, die aus Antiglaukomatika, Entzündungshemmern, Immunsuppressiva, Virostatika, antibakteriellen Mitteln, Antimykotika oder Antioxidantien besteht, und/oder einen oder mehrere Wirkstoffe in therapeutisch wirksamer Menge gegen trockene Augen, wie beispielsweise Polyacrylsäurederivate, Carbomere, Trehalose, Hyaluronsäure oder deren Salze, Povidon, Polyvinylalkohol, Cellulosederivate, Chitosan-Derivate, Phospholipide und Triglyceride, aufweist.

## Claims

1. A dispenser containing an ophthalmic product that can be administered in the form of drips,
the dispenser including a reservoir (1) having a wall (2) defining an internal space and suitable for deforming when pressure is applied to the reservoir (1) by a user of the dispenser and for spontaneously returning to its original shape upon release of said pressure,
the reservoir (1) further including a flexible bag (3) in the internal space defined by the wall (2) and in which the ophthalmic product is contained, the dispenser including:
- an air inlet means (11) for receiving air between the wall (2) of the reservoir (1) and the flexible bag (3),
- a head attached onto a neck (6) of the reservoir (1) and including a drip end piece (8) having a dispensing opening, and
- a unidirectional valve (10) interposed between the flexible bag (3) and the dispensing opening, the unidirectional valve being configured to prevent air from outside the dispenser from entering into the flexible bag (3), such that once the ophthalmic product has been dispensed, the dispensed volume of ophthalmic product is compensated by a corresponding volume of air being received between the wall (2) of the reservoir (1) and the flexible bag (3) via the air inlet means (11),
**characterised in that** the ophthalmic product is a viscous ophthalmic product (5), that is to say having a viscosity greater than or equal to 200 cP and is sterile and free from preservatives,
and **in that**, the dispenser including internal surfaces located between the unidirectional valve (10) and the dispensing opening, all of said internal surfaces having a bactericidal treatment and/or a treatment that reduces the wettability thereof.

2. The dispenser containing a viscous ophthalmic product according to claim 1, wherein the flexible bag (3) is formed by delamination of an internal layer of the reservoir (1).

3. The dispenser containing a viscous ophthalmic product according to claim 1, wherein the flexible bag (3) is a retractable flexible bag.

4. The dispenser containing a viscous ophthalmic product according to one of claims 1 to 3, wherein the air inlet means (11) is formed by a hole (12) in the wall (2) of the reservoir (1) adapted to be closed by a finger (13) of the user.

5. The dispenser containing a viscous ophthalmic product according to one of claims 1 to 3, wherein the air inlet means (11) includes a unidirectional valve (22), configured to allow receiving air between the wall (2) of the reservoir (1) and the flexible bag (3) and to prevent the air present between the wall (2) of the reservoir (1) and the flexible bag (3) from escaping to the outside of the reservoir (1).

6. The dispenser containing a viscous ophthalmic product according to one of the preceding claims, wherein the bactericidal treatment is based on silver ions or zinc ions.

7. The dispenser containing a viscous ophthalmic product according to claim 6, wherein the bactericidal treatment includes silver ions or zinc ions included in the mass of the elements forming said internal surfaces located between the unidirectional valve and the dispensing opening.

8. The dispenser containing a viscous ophthalmic product according to one of claims 1 to 7, wherein the bactericidal treatment includes a bactericidal surface coating.

9. The dispenser containing a viscous ophthalmic product according to one of the preceding claims, wherein an external surface (17) of the unidirectional valve (10) oriented towards the dispensing opening, also has the bactericidal treatment.

10. The dispenser containing a viscous ophthalmic product according to one of the preceding claims wherein the treatment that reduces the wettability of the internal surfaces located between the unidirectional valve (10) and the dispensing opening is a treatment of the fluorinated or silicone plasma treatment type.

11. The dispenser containing a viscous ophthalmic product according to one of claims 1 to 9 wherein the treatment that reduces the wettability of the internal surfaces located between the unidirectional valve (10) and the dispensing opening is a laser surface texturing.

12. The dispenser containing a viscous ophthalmic product according to one of the preceding claims including a removable cap (18), attached to the dispensing head.

13. The dispenser containing a viscous ophthalmic product according to one of the preceding claims wherein the unidirectional valve (10) includes an element deformable under the effect of excess pressure inside the reservoir of the dispenser, or includes a ball check-valve (24).

14. The dispenser containing a viscous ophthalmic product according to one of the preceding claims, wherein said viscous ophthalmic product (5) includes one or more active ingredients in a therapeutically effective amount selected from the group of anti-glaucoma, antiinflammatories, immunosuppressants, antivirals, antibacterials, antifungals or antioxidants, and/or one or more active ingredients in a therapeutically effective amount against dry eyes such as polyacrylic acid derivatives, carbomers, trehalose, hyaluronic acid or its salts, povidone, polyvinyl alcohol, cellulose derivatives, chitosan derivatives, phospholipids, triglycerides.
